# EUROPÄISCHE PATENTANMELDUNG

(11) **EP 3 379 187 A1**
(43) Veröffentlichungstag der Anmeldung: **26.09.2018**
(21) Anmeldenummer: 17020274.1
(22) Anmeldetag: 29.06.2017
(51) Int. Cl.: F25J 3/06

(54) **ERZEUGUNG EINES EINSATZSTROMS FÜR EINE ALKAN-DEHYDRIERUNG**

(30) Priorität: 21.03.2017 DE 102017002737
(71) Anmelder: Linde Aktiengesellschaft, 80331 München (DE)
(72) Erfinder: Eberl, Christian, 85586 Poing (DE)

(57) **Zusammenfassung**

Die Erfindung betrifft ein Verfahren sowie eine Vorrichtung zur Erzeugung eines kombinierten Einsatzstroms (6) für eine Alkan-Dehydrierung (R), bei dem ein weitgehend aus einem Alkan bestehender Flüssigeinsatz (1) abgekühlt und nach Kombination mit einer Wasserstofffraktion (4) verdampft und angewärmt wird, um den kombinierten Einsatzstrom (6) zu bilden, wobei das Wasserstoff sowie ein Alken umfassende, in der Alkan-Dehydrierung (R) gewonnene Dehydrier-Produkt (7) gegen den nach seiner Abkühlung zu verdampfenden und anzuwärmenden, weitgehend aus einem Alkan bestehenden Flüssigeinsatz abgekühlt wird, um das Alken in einer ersten Flüssigphase (9) auszukondensieren und eine erste wasserstoffreiche Gasphase (10) zu erzeugen, aus der nach weiterer Abkühlung die Wasserstofffraktion (4) erhalten wird. Kennzeichnend hierbei ist, dass für den Prozess benötigte Kälte über ein unabhängig geführtes Kältemittel (25) bereitgestellt wird.

## Beschreibung

Die Erfindung betrifft ein Verfahren zur Erzeugung eines kombinierten Einsatzstroms für eine Alkan-Dehydrierung, bei dem ein weitgehend aus einem Alkan bestehender Flüssigeinsatz abgekühlt und nach Kombination mit einer Wasserstofffraktion verdampft und angewärmt wird, um den kombinierten Einsatzstrom zu bilden, wobei das Wasserstoff sowie ein Alken umfassende, in der Alkan-Dehydrierung gewonnene Dehydrier-Produkt gegen den nach seiner Abkühlung zu verdampfenden und anzuwärmenden, weitgehend aus einem Alkan bestehenden Flüssigeinsatz abgekühlt wird, um das Alken in einer ersten Flüssigphase auszukondensieren und eine erste wasserstoffreiche Gasphase zu erzeugen, aus der nach weiterer Abkühlung die Wasserstofffraktion erhalten wird.

Weiterhin betrifft die Erfindung eine Vorrichtung zur Durchführung des erfindungsgemäßen Verfahrens.

Ein Verfahren der gattungsgemäßen Art wird beispielsweise im sog. Olefelx™-Prozess eingesetzt, der insbesondere zur Erzeugung von Propylen aus Propan bzw. von Butylen aus Butan verwendet wird. Die zur Durchführung des Prozesses dienende Vorrichtung umfasst einen Dehydrier-Reaktor sowie eine kryogene Behandlungseinrichtung, die in einer wärmeisolierenden Einhausung, der sog. Cold Box, angeordnet ist. Im Dehydrier-Reaktor, dem ein das jeweilige Alkan sowie Wasserstoff enthaltender, als kombinierter Einsatz bezeichneter Stoffstrom zugeführt wird, wird das Alkan mit Hilfe eines geeigneten Katalysators zum Alken umgesetzt, wobei ein gasförmiges, Wasserstoff und das Alken enthaltendes Dehydrier-Produkt entsteht.

Die kryogene Behandlungseinrichtung dient zur Zerlegung des Dehydrier-Produkts in ein Wasserstoffprodukt und eine kohlenwasserstoffreiche, das erzeugte Alken umfassende Flüssigfraktion, das sog. Flüssigprodukt. Weiterhin wird im kryogenen Behandlungsteil aus einem im Wesentlichen aus dem jeweiligen Alkan bestehenden Flüssigeinsatz und einem Teil des aus dem Dehydrier-Produkt abgetrennten Wasserstoffs der kombinierte Einsatzstrom mit einem vorgegebenen Verhältnis von Wasserstoff zu Kohlenwasserstoff gemischt.

Der Wasserstoff im kombinierten Einsatzstrom wirkt als Moderator für die chemische Gleichgewichtsreaktion im Dehydrier-Reaktor. Je mehr Wasserstoff im Einsatzgas vorhanden ist, desto geringer ist der Umsatz der Dehydrier-Reaktion von Alkan zu Alken. Daher wird in der Praxis ein möglichst niedriges Verhältnis von Wasserstoff zu Kohlenwasserstoff im kombinierten Einsatz angestrebt, das gewöhnlich zwischen 0,4 und 1,0 liegt. Gleichzeitig erhöht sich aber die Gefahr einer Beschädigung des Katalysatormaterials im Dehydrier-Reaktor mit sinkendem Wasserstoffgehalt im kombinierten Einsatzstrom.

Ein Großteil der für den Prozess benötigten Kälte wird durch die arbeitsleistende Entspannung einer bei der Zerlegung des Dehydrier-Produkts anfallenden wasserstoffreichen Gasphase über einen ersten Expander bereitgestellt. Die Entspannung erfolgt dabei auf einen Druck, der - bis auf Leitungsverluste - dem Abgabedruck des Wasserstoffprodukts entspricht. Das bei der Entspannung anfallende zweiphasige Stoffgemisch wird in einem Abscheider in eine kohlenwasserstoffreiche Flüssig- und eine wasserstoffreiche Gasphase getrennt, von der ein größerer erster Teil nach Anwärmung gegen abzukühlende Verfahrensströme als Wasserstoffprodukt abgegeben wird, während ein kleinerer zweiter Teil über einen zweiten Expander arbeitsleistend weiter entspannt wird, um die für den Prozess erforderliche Spitzenkälte zu liefern. Auch bei dieser zweiten Entspannung fällt ein zweiphasiges Stoffgemisch an, das in einem weiteren Abscheider in eine weitere kohlenwasserstoffreiche Flüssigphase und eine Wasserstofffraktion getrennt wird, die anschließend als sog. Recycle Gas vollständig dem gegen anzuwärmende Verfahrensströme abgekühlten Flüssigeinsatz zugemischt wird, um den kombinierten Einsatzstrom zu erzeugen.

Die Verwendung verbesserter Katalysatoren im Dehydrier-Reaktor erlaubt es, das Wasserstoff/Kohlenwasserstoff-Verhältnis im kombinierten Einsatzstrom bis auf ca. 0,15 abzusenken, so dass der Umsatz der Dehydrier-Reaktion gesteigert wird und mehr Alkene im Dehydrier-Produkt enthalten sind. Durch die geringere Menge an Wasserstoff im kombinierten Einsatzstrom steht dann allerdings nicht genügend wasserstoffreiches Gas zur Verfügung, um durch dessen arbeitsleistende Entspannung genügend Kälte für die Abkühlung und Kondensation des Dehydrier-Produkts zu erzeugen.

Aufgabe der vorliegenden Erfindung ist es daher, ein Verfahren und eine Vorrichtung der gattungsgemäßen Art anzugeben, die es erlauben, auch bei niedrigen Wasserstoffgehalten im kombinierten Einsatzstrom ausreichend Kälte für die Kondensation des Dehydrier-Produkts bereitzustellen.

Die gestellte Aufgabe wird verfahrensseitig erfindungsgemäß dadurch gelöst, dass für den Prozess benötigte Kälte über ein unabhängig geführtes Kältemittel bereitgestellt wird.

Die unabhängige Führung des Kältemittels ist dabei so zu verstehen, dass zwischen dem Kältemittel und den übrigen am Prozess beteiligten Stoffströmen zwar Wärme jedoch keine Materie ausgetauscht werden kann. Weder wird das Kältemittel aus einem der übrigen am Verfahren beteiligten Stoffströmen gebildet, noch im Prozess mit einem von diesen vereinigt.

Zweckmäßigerweise wird das Kältemittel in einem geschlossenen Kühlkreislauf geführt, wobei es nach Verdampfung und Anwärmung gegen einen oder mehrere am Prozess beteiligte Stoffströme verdichtet, abgekühlt, kondensiert und kälteleistend entspannt wird. Als Kältemittel eignet sich hierbei insbesondere Ethylen, mit dem Kälte problemlos auf einem Temperaturniveau von -100°C erzeugt werden kann.

Als Alternative zu Ethylen kann auch ein auf den Prozess abgestimmtes, aus mehreren Komponenten bestehendes Kältemittel verwendet werden. Als mögliche Komponenten eines solchen Kältemittels sollen Kohlenwasserstoffe, insbesondere C1- bis C5-Kohlenwasserstoffe, sowie Stickstoff erwähnt werden. Weiterhin kann reiner Stickstoff als Kältemittel in dem geschlossenen Kühlkreislauf eingesetzt werden.

Möglich ist es auch, eine kryogene Flüssigkeit wie beispielsweise Flüssigstickstoff als Kältemittel in einem offenen Kreislauf zu fahren, wobei die kryogene Flüssigkeit nach Verdampfung und Anwärmung gegen am Prozess beteiligte Stoffströme in die Atmosphäre abgegeben wird.

Das erfindungsgemäße Verfahren weiterbildend wird vorgeschlagen, die aus dem Dehydrier-Produkt gewonnene erste wasserstoffreiche Gasphase soweit abzukühlen, dass ein zweiphasiges Stoffgemisch entsteht, das in eine zweite alkenreiche Flüssig- und eine zweite wasserstoffreiche Gasphase getrennt wird, von der ein Teilstrom lediglich über ein Drosselorgan entspannt wird, um die für die Erzeugung des kombinierten Einsatzstroms benötigte Wasserstofffraktion zu erhalten.

Eine andere Variante des erfindungsgemäßen Verfahrens sieht dagegen vor, dass die gesamte zweite wasserstoffreiche Gasphase über einen Expander kälteleistend entspannt wird, um ein weiteres zweiphasiges Stoffgemisch zu erhalten, das in eine weitere alkenreiche Flüssig- und eine weitere wasserstoffreiche Gasphase getrennt wird, von der ein erster Teil lediglich über ein Drosselorgan entspannt wird, um die für die Erzeugung des kombinierten Einsatzstroms benötigte Wasserstofffraktion zu erhalten.

Mit besonderem Vorzug kann das erfindungsgemäße Verfahren bei der Erzeugung von Propylen aus Propan oder von Butylen aus Butan eingesetzt werden, wobei seine Verwendung bei der Herstellung anderer Alkene nicht ausgeschlossen sein soll.

Weiterhin betrifft die Erfindung eine Vorrichtung zur Erzeugung eines kombinierten Einsatzstroms für einen Dehydrier-Reaktor, mit wenigstens zwei Wärmetauschern zur Abkühlung eines weitgehend aus einem Alkan bestehenden Flüssigeinsatzes sowie zur partiellen Kondensation eines aus dem Dehydrier-Reaktor zuführbaren, Wasserstoff sowie ein Alken umfassenden Dehydrier-Produkts gegen den nach seiner Abkühlung und der Kombination mit einer Wasserstofffraktion zu verdampfenden und anzuwärmenden, weitgehend aus einem Alkan bestehenden Flüssigeinsatz, und wenigstens einem Abscheider, mit dem das bei der partiellen Kondensation des Dehydrier-Produkts anfallende zweiphasige Stoffgemisch in ein Flüssigkeitsgemisch, das das Alken enthält, sowie ein erstes wasserstoffreiches Gasgemisch getrennt werden kann, aus dem nach weiterer Abkühlung die Wasserstofffraktion erhältlich ist.

Die gestellte Aufgabe wird vorrichtungsseitig erfindungsgemäß dadurch gelöst, dass wenigstens einer der Wärmetauscher Teil eines Kühlkreislaufs ist, durch den ein Kältemittel unabhängig geführt werden kann.

Vorzugsweise ist der Kühlkreislauf geschlossen und mit Einrichtungen ausgeführt, über die das im Wärmetauscher gegen einen oder mehrere am Prozess beteiligte Stoffströme verdampfte und/oder angewärmte Kältemittel verdichtet, abgekühlt, kondensiert und kälteleistend entspannt werden kann. Besonders bevorzugt weist der geschlossene Kältekreislauf einen Verdichter und einen Expander oder eine Kombination beider Maschinen, einen sog. Compander, auf.

Ebenfalls möglich ist ein offener Kühlkreislauf, der mit einem Speicherbehälter verbunden ist, aus dem eine kryogene Flüssigkeit wie beispielsweise Flüssigstickstoff entnommen werden kann, um als Kältemittel durch den wenigstes einen Wärmetauscher geführt und anschließend etwa in einer Fackel entsorgt zu werden.

Weiterhin bevorzugt umfasst die erfindungsgemäße Vorrichtung ein Drosselorgan sowie einen zweiten Abscheider, in dem die erste wasserstoffreiche Gasphase nach ihrer Abkühlung in eine zweite alkenreiche Flüssig- und eine zweite wasserstoffreiche Gasphase getrennt werden kann, von der ein erster Teil über das Drosselorgan entspannbar ist, um die Wasserstofffraktion für die Mischung mit dem weitgehend aus einem Alkan bestehenden Flüssigeinsatz zu bilden.

Eine Ausgestaltung der erfindungsgemäßen Vorrichtung sieht die Anordnung eines dritten Abscheiders sowie eines Expanders zwischen dem zweiten Abscheider und dem Drosselorgan vor, so dass die zweite wasserstoffreiche Gasphase aus dem zweiten Abscheider kälteleistend über den Expander entspannt und anschließend im dritten Abscheider in eine weitere alkenreiche Flüssig- und eine weitere wasserstoffreiche Gasphase getrennt werden kann, von der ein erster Teil über das Drosselorgan entspannbar ist, um die Wasserstofffraktion für die Mischung mit dem weitgehend aus einem Alkan bestehenden Flüssigeinsatz zu bilden.

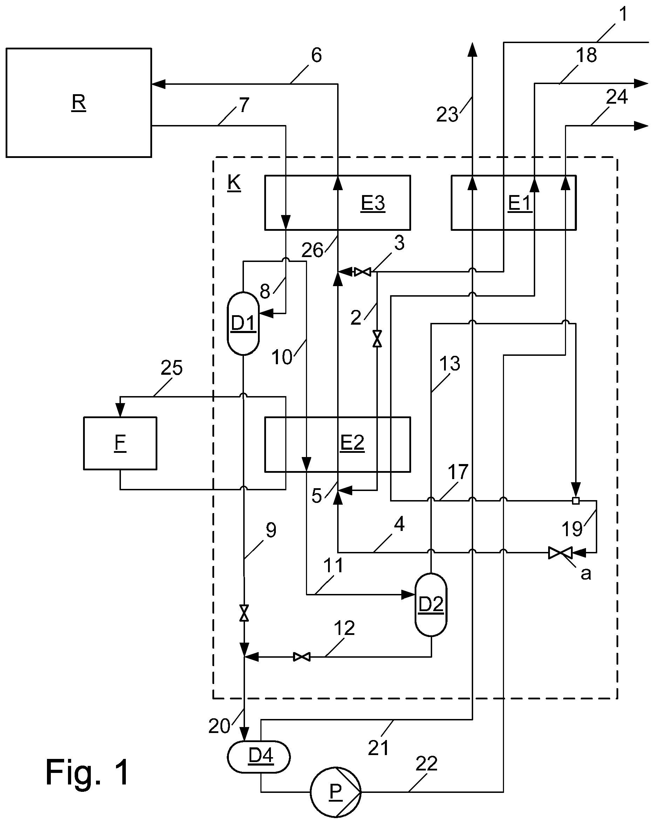

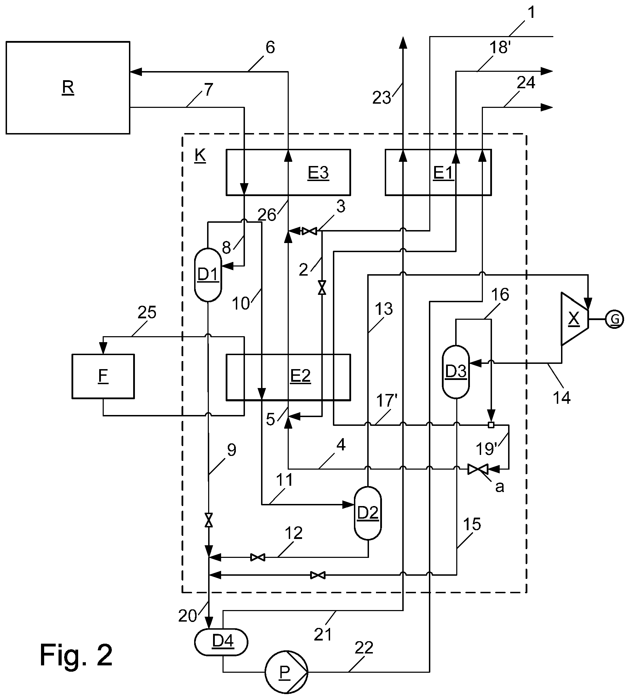

Im Folgenden soll die Erfindung anhand zweier in den **Figuren 1** und **2** schematisch dargestellter Ausführungsbeispiele näher erläutert werden.

In beide Figuren, von denen jede eine besonders bevorzugte Variante der Erfindung zeigt, wie sie insbesondere bei der Erzeugung von Propylen aus Propan eingesetzt werden kann, sind gleiche Anlagenteile und Stoffströme mit den gleichen Bezugszeichen versehen.

Über Leitung 1 wird ein weitgehend aus einem Alkan bestehender Flüssigeinsatz in die kryogene Behandlungseinrichtung K eingeleitet und nach einer ersten Abkühlung im ersten Wärmetauscher E1 in die beiden Teilströme 2 und 3 aufgeteilt, von denen der eine 2 im zweiten Wärmetauscher E2 weiter abgekühlt wird. Anschließend wird der weiter abgekühlte Teilstrom 2 mit einer Wasserstofffraktion 4 zum Stoffstrom 5 kombiniert, der nach Verdampfung und Anwärmung im zweiten Wärmetauscher E2 mit dem zweiten Teilstrom 3 des Flüssigeinsatzes 1 zum Stoffstrom 26 vereinigt wird, dem im dritten Wärmtauscher E3 Wärme zugeführt wird, um schließlich gasförmig den kombinierten Einsatzstroms 6 für den Dehydrier-Reaktor R zur erhalten. Die Menge der Wasserstofffraktion 4 wird dabei so geregelt, dass der kombinierte Einsatzstrom 6 mit einem vorgegebenen Wasserstoff/Kohlenwasserstoff-Verhältnis erzeugt wird.

Im Dehydrier-Reaktor R wird das im kombinierten Einsatzstrom 6 enthaltene Alkan mit katalytischer Unterstützung dehydriert, um ein vorwiegend aus dem zugehörigen Alken und Wasserstoff bestehendes Dehydrier-Produkt 7 zu gewinnen, das in die kryogene Behandlungseinrichtung K zurückgeführt wird. Hier wird es im dritten Wärmetauscher E3 gegen den Stoffstrom 26 abgekühlt, wobei ein erster zweiphasiger Stoffstrom 8 entsteht, der im ersten Abscheider D1 in eine erste kohlenwasserstoffreiche Flüssig- 9 und eine erste wasserstoffreiche Gasphase 10 getrennt wird. Aus der ersten wasserstoffreichen Gasphase 10 wird durch weitere Abkühlung im Wärmetauscher E2 ein zweiter zweiphasiger Stoffstrom 11 erzeugt, der im zweiten Abscheider D2 in eine zweite kohlenwasserstoffreiche Flüssig- 12 und eine zweite wasserstoffreiche Gasphase 13 zerlegt wird.

Die beiden kohlenwasserstoffreichen Flüssigphasen 9 und 12 werden entspannt, zum Flüssigstrom 20 zusammengefasst und in einen außerhalb der kryogenen Behandlungseinrichtung K angeordneten Behälter D4 eingeleitet, wobei leichte Komponenten in die Gasfraktion 21 übergehen und eine weitgehend aus dem Alken bestehende, andere Kohlenwasserstoffe umfassende Flüssigfraktion 22 erhalten wird. Der Druck der Flüssigfraktion 22 wird mit Hilfe der Pumpe P angehoben, bevor sie wie die Gasfraktion 21 in die kryogene Behandlungseinrichtung K zurückgeführt und im Wärmetauscher E1 gegen den abzukühlenden Flüssigeinsatz 1 angewärmt wird. Schließlich werden die angewärmten Phasen als Flash Gas 23 bzw. Flüssigprodukt 24 abgegeben.

Für die Zerlegung des Dehydrier-Produkts 7 benötigte Kälte wird über die Kältequelle F zur Verfügung gestellt, deren z.B. mit Ethylen als Kältemittel betriebener Kühlkreislauf 25 hierzu über den zweiten Wärmetauscher E2 der kryogenen Behandlungseinrichtung K geführt wird.

Im Ausführungsbeispiel der Figur 1 wird ein erster Teil 17 der zweiten wasserstoffreichen Gasphase 13 in den Wärmetauschern E2 und E1 gegen abzukühlende Verfahrensströme angewärmt und als Wasserstoffprodukt 18 weitergeführt, während der zweite Teil 19 zur Bildung der Wasserstofffraktion 4 dient, wozu er lediglich über das Drosselorgan a auf den zur Bildung des kombinierten Einsatzstroms 6 erforderlichen Druck entspannt wird.

Bei der in Figur 2 gezeigten Variante wird die zweite wasserstoffreiche Gasphase 13 zur weiteren Auftrennung und der Erzeugung von Prozesskälte über den mit einem Generator G gekoppelten Expander X arbeitsleistend entspannt, wobei sich ein dritter zweiphasiger Stoffstrom 14 bildet, der in einem dritten Abscheider D3 in eine dritte kohlenwasserstoffreiche Flüssig- 15 und eine Produktreinheit aufweisende dritte zweite wasserstoffreiche Gasphase 16 getrennt wird, von der nachfolgend ein erster Teil 17' in den Wärmetauschern E2 und E1 gegen abzukühlende Verfahrensströme angewärmt und als Wasserstoffprodukt 18' weitergeführt wird, während der zweite Teil 19' zur Bildung der Wasserstofffraktion 4 dient, wozu er lediglich über das Drosselorgan a auf den zur Bildung des kombinierten Einsatzstroms 6 erforderlichen Druck entspannt wird. Die dritte kohlenwasserstoffreiche Flüssigphase 15 wird gemeinsam mit dem Flüssigstrom 20 in den Behälter D4 eingeleitet.

## Patentansprüche

1. Verfahren zur Erzeugung eines kombinierten Einsatzstroms (6) für eine Alkan-Dehydrierung (R), bei dem ein weitgehend aus einem Alkan bestehender Flüssigeinsatz (1) abgekühlt und nach Kombination mit einer Wasserstofffraktion (4) verdampft und angewärmt wird, um den kombinierten Einsatzstrom (6) zu bilden, wobei das Wasserstoff sowie ein Alken umfassende, in der Alkan-Dehydrierung (R) gewonnene Dehydrier-Produkt (7) gegen den nach seiner Abkühlung zu verdampfenden und anzuwärmenden, weitgehend aus einem Alkan bestehenden Flüssigeinsatz abgekühlt wird, um das Alken in einer ersten Flüssigphase (9) auszukondensieren und eine erste wasserstoffreiche Gasphase (10) zu erzeugen, aus der nach weiterer Abkühlung die Wasserstofffraktion (4) erhalten wird, **dadurch gekennzeichnet, dass** für den Prozess benötigte Kälte über ein unabhängig geführtes Kältemittel (25) bereitgestellt wird.

2. Verfahren nach Anspruch 1, **dadurch gekennzeichnet, dass** als Kältemittel (25) Ethylen oder Stickstoff oder ein Stoffgemisch eingesetzt wird, dessen wenigstens zwei Komponenten aus C1- bis C5-Kohlenwasserstoffen sowie Stickstoff ausgewählt sind.

3. Verfahren nach einem der Ansprüche 1 oder 2, **dadurch gekennzeichnet, dass** die erste wasserstoffreiche Gasphase (10) durch Abkühlung zu einem zweiphasigen Stoffgemisch (11) umgesetzt und in eine zweite alkenreiche Flüssigphase (12) sowie eine zweite wasserstoffreiche Gasphase (13) getrennt wird, von der ein Teilstrom (19) abgezweigt und lediglich über ein Drosselorgan (a) entspannt wird, um die Wasserstofffraktion (4) zu erhalten.

4. Verfahren nach einem der Ansprüche 1 oder 2, **dadurch gekennzeichnet, dass** die erste wasserstoffreiche Gasphase (10) durch Abkühlung zu einem zweiphasigen Stoffgemisch (11) umgesetzt und in eine zweite alkenreiche Flüssigphase (12) sowie eine zweite wasserstoffreiche Gasphase (13) getrennt wird, die über einen Expander (X) kälteleitend entspannt wird, um ein weiteres zweiphasiges Stoffgemisch (14) zu erhalten, das in eine weitere alkenreiche Flüssig- (15) und eine weitere wasserstoffreiche Gasphase (16) getrennt wird, von der ein erster Teil (19') lediglich über ein Drosselorgan entspannt wird, um die Wasserstofffraktion (4) zu erhalten.

5. Verfahren nach einem der Ansprüche 1 bis 4, **dadurch gekennzeichnet, dass** es zur Erzeugung eines kombinierten Einsatzstroms (6) für eine Propan- oder eine Butan-Dehydrierung (R) eingesetzt wird.

6. Vorrichtung (K) zur Erzeugung eines kombinierten Einsatzstroms (6) für einen Dehydrier-Reaktor (R), mit wenigstens zwei Wärmetauschern (E1, E2) zur Abkühlung eines weitgehend aus einem Alkan bestehenden Flüssigeinsatzes (1) sowie zur partiellen Kondensation eines aus dem Dehydrier-Reaktor (R) zuführbaren, Wasserstoff sowie ein Alken umfassenden Dehydrier-Produkts (7) gegen den nach seiner Abkühlung und der Kombination mit einer Wasserstofffraktion (4) zu verdampfenden und anzuwärmenden, weitgehend aus einem Alkan bestehenden Flüssigeinsatz, und wenigstens einem Abscheider (D1), mit dem das bei der partiellen Kondensation des Dehydrier-Produkts (7) anfallende zweiphasige Stoffgemisch (8) in ein Flüssigkeitsgemisch (9), das das Alken enthält, sowie ein erstes wasserstoffreiches Gasgemisch (10) getrennt werden kann, aus dem nach weiterer Abkühlung die Wasserstofffraktion (4) erhältlich ist, **dadurch gekennzeichnet, dass** wenigstens einer der Wärmetauscher (E2) Teil eines Kühlkreislaufs (25) ist, durch den ein Kältemittel unabhängig geführt werden kann.

7. Vorrichtung nach Anspruch 6, **dadurch gekennzeichnet, dass** der Kühlkreislauf (25) geschlossen oder offen ausgeführt ist.

8. Vorrichtung nach einem der Ansprüche 6 oder 7, **dadurch gekennzeichnet, dass** der Kältekreislauf einen Verdichter und einen Expander oder einen Compander aufweist.

9. Vorrichtung nach einem der Ansprüche 6 oder 8, **dadurch gekennzeichnet, dass** sie ein Drosselorgan (a) sowie einen zweiten Abscheider (D2) umfasst, in dem das erste wasserstoffreiche Gasgemisch (10) nach seiner Abkühlung in eine zweite alkenreiche Flüssigphase (12) und eine zweite wasserstoffreiche Gasphase (13) getrennt werden kann, von der ein Teilstrom über das Drosselorgan (a) entspannbar ist, um die Wasserstofffraktion (4) zu bilden.

10. Vorrichtung nach Anspruch 9, **dadurch gekennzeichnet, dass** zwischen dem zweiten Abscheider (D2) und dem Drosselorgan (a) ein dritter Abscheider (D3) sowie ein Expander (X) angeordnet sind, so dass die zweite wasserstoffreiche Gasphase (13) aus dem zweien Abscheider (D2) kälteleistend über den Expander (X) entspannt und anschließend im dritten Abscheider (D3) in eine weitere alkenreiche Flüssig- und eine weitere wasserstoffreiche Gasphase (16) getrennt werden kann, von der ein Teilstrom (19') über das Drosselorgan (a) entspannbar ist, um die erste Wasserstofffraktion (4) zu bilden.
